# EUROPÄISCHE PATENTSCHRIFT

(11) **EP 0 543 244 B1**
(45) Veröffentlichungstag und Bekanntmachung des Hinweises auf die Patenterteilung: **20.08.1997**
(21) Anmeldenummer: 92119127.6
(22) Anmeldetag: 09.11.1992
(51) Int. Cl.: C07C 25/24, C09K 19/18, C09K 19/34, C09K 19/30, C07C 43/225, C07C 255/50, C07D 213/26, C07D 239/26, C07D 319/06, G02F 1/13

(54) **Fluorsubstituierte Tolane und sie enthaltende flüssigkristalline Gemische**
Fluoro substituted tolans and liquid crystal mixtures containing them
Tolanes fluoro substitués et mélanges de cristaux liquides les contenant

(30) Priorität: 19.11.1991 CH 3375/91; 18.06.1992 CH 1918/92
(43) Veröffentlichungstag der Anmeldung: 26.05.1993
(73) Patentinhaber: MERCK PATENT GmbH, 64293 Darmstadt (DE)
(72) Erfinder: Buchecker, Richard, CH-8008 Zürich (CH); Schadt, Martin, CH-4411 Seltisberg (CH)

(56) Entgegenhaltungen:
- WO-A-88/07514
- DE-A- 4 105 742
- CHEMICAL ABSTRACTS Columbus, Ohio, US; vol. 116, no. 72519n & JP-A-03 206 055

## Beschreibung

Die vorliegende Erfindung betrifft neue Verbindungen mit mehrfach fluorierter Tolanstruktur, deren Herstellung, flüssigkristalline Gemische, die solche Verbindungen enthalten, sowie die Verwendung dieser Verbindungen bzw. Gemische für elektro-optische Zwecke.

Der Anmelder hat sich unter Bezugnahme auf DE-A 4 105 742, freiwillig eingeschränkt und gesonderte Papentansprüche für Deutschland vorgelegt.

Flüssige Kristalle werden vor allem als Dielektrika in Anzeigevorrichtungen verwendet, da die optischen Eigenschalten solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super twisted nematic"), SBE-Zellen ("super birefringence effect") und OMI-Zellen ("optical mode interference"). Für Displays mit hohem Informationsgehalt sind in letzter Zeit neben den passiv angesteuerten, multiplexierten besonders die aktiv angesteuerten, z.B. TFT-Zellen ("thin film transistor") wichtig geworden. Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische, photochemische und thermische Stabilität und eine gute Stabilität gegenüber elektrischen Feldern besitzen. Ferner sollten sie über einen möglichst breiten Bereich eine geeignete Mesophase aufweisen (beispielsweise für die oben genannten Zellen eine nematische bzw. eine cholesterische Phase), aber trotzdem ausreichend niedere Viskosität besitzen und in den Zellen kurze Ansprechzeiten, tiefe Schwellenspannungen und einen hohen Kontrast ermöglichen. Weitere Eigenschaften, wie die elektrische Leitfähigkeit, die dielektrische Anisotropie und die optische Anisotropie, müssen je nach Anwendungsgebiet und Zellentyp unterschiedlichen Anforderungen genügen. Beispielsweise sollten Materialien für die Zellen mit verdrillt nematischer Struktur eine möglichst hohe positive dielektrische Anisotropie und gleichzeitig eine möglichst geringe Leitfähigkeit aufweisen. Diese letztere Eigenschaft ist vorallem für TFT-Zellen von besonderer Wichtigkeit. Leider führen aber Komponenten mit höherer dielektrischer Anisotropie infolge ihres besseren Lösungsvermögens für ionische Verunreinigungen meist zu einer erhöhten Leitfähigkeit in Mischungen. Es werden daher Komponenten gesucht, die sich durch eine möglichst hohe dielektrische Anisotropie bei gleichzeitig möglichst geringer Leitfähigkeit auszeichnen.

Gegenstand der Erfindung sind Verbindungen der allgemeinen Formel 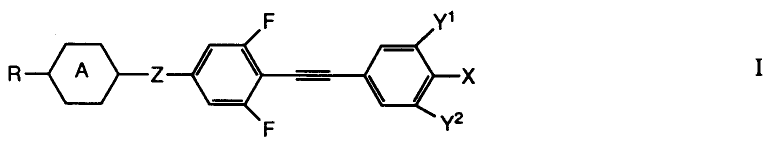 worin
- Ring A: 1,4-Phenylen, Pyridin-2,5-diyl, Pyrimidin-2,5-diyl, trans-1,4-Cyclohexylen oder trans 1,3-Dioxan-2,5-diyl darstellt;
- Z: eine einfache Kovalenzbindung, -CH₂CH₂-, -OCH₂-, -CH₂O-, -(CH₂)₄-, -O(CH₂)₃-, -(CH₂)₃O-, oder, falls Ring A einen gesättigten Ring darstellt, auch die trans-Form von -CH=CH(CH₂)₂- oder -CH=CHCH₂O- bedeutet;
- X: Fluor, Chlor, Cyano, -CF₃, -OCF₃, -OCHF₂, Alkyl, Alkoxy oder Alkoxyalkyl mit 1 bis 6 Kohlenstoffatomen bedeutet;
- Y^{1,} Y²: unabhängig voneinander Fluor oder Wasserstoff bedeuten ; und
- R: Alkyl, Alkoxy, Alkenyl oder Alkenyloxy mit 1 bzw. 2 bis 12 Kohlenstoffatomen bedeutet, in welchen eine oder zwei miteinander nicht benachbarte CH₂-Gruppen durch Sauerstoff und/oder eines oder mehrere Wasserstoffatome durch Fluoratome ersetzt sein können.

Die erfindungsgemässen Verbindungen sind Flüssigkristalle mit ausgeprägter nematischer Phase, hoher optischer und dielektrischer Anisotropie bei relativ niedriger Rotationsviskosität und führen zu vergleichsweise niedrigen Schwellenspannungen und kurzen Ansprechzeiten. Trotz der hohen dielektrischen Anisotropie ist die Leitfähigkeit relativ niedrig. Zudem ist der Klärpunkt trotz mehrfacher lateraler Substitution überraschend hoch bei vergleichsweise niedrigem Schmelzpunkt und kleiner Schmelzenthalpie. Durch die geeignete Wahl eines gesättigten oder aromatischen Rings für A kann die relativ hohe optische Anisotropie wunschgemäss erniedrigt oder noch weiter erhöht werden.

Die erfindungsgemässen Verbindungen sind in Mischungen sehr gut und in breiten Konzentrationsbereichen löslich. Sie eignen sich besonders zur Anwendung in Mischungen, die bei tiefer Schwellenspannung eine tiefe Leitfähigkeit und gleichzeitig eine vergleichsweise hohe optische Anisotropie aufweisen sollten, beispielsweise für TN-, STN- oder TFT-Zellen mit geringer Schichtdicke oder mit besonders hohem Kontrast, wie sie beispielsweise für Projektionsdisplays benötigt werden.

Der Ausdruck "gesättigter Ring" umfasst im Rahmen der vorliegenden Erfindung trans-1,4-Cyclohexylen oder trans-1,3-Dioxan-2,5-diyl.

In den Verbindungen I, worin A einen heterocyclischen Ring wie Pyridin-2,5-diyl, Pyrimidin-2,5-diyl oder trans-1,3-Dioxan-2,5-diyl bedeutet, sind die Heteroatome im Ring ausschliesslich so angeordnet, dass sie eine zur Verknüpfungsstelle mit Z benachbarte Stellung einnehmen.

Das Brückenglied Z bedeutet bevorzugt eine einfache Kovalenzbindung, -CH₂CH₂-, -OCH₂- oder -CH₂O-, besonders bevorzugt jedoch eine einfache Kovalenzbindung oder -CH₂CH₂-.

Bevorzugte Untergruppen der Verbindungen der Formel I sind somit die Verbindungen der allgemeinen Formeln 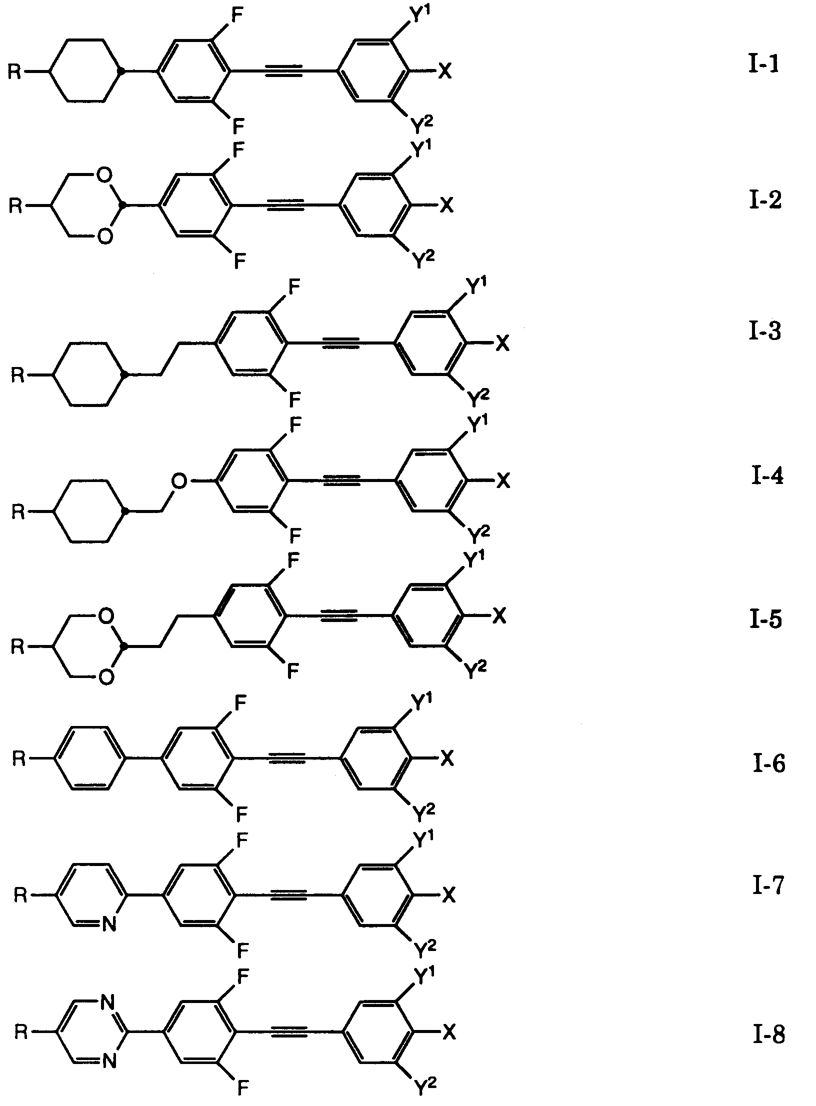 worin R, X, Y¹ und Y² die für Formel I definierten Bedeutungen haben.

Der Rest R in den Formeln I und I-1 bis I-8 kann 1 bzw. 2 bis 12, bevorzugt 1 bzw. 2 bis 6 Kohlenstoffatome aufweisen. Er kann prinzipiell geradkettig oder verzweigt sein, bevorzugt jedoch geradkettig. Im Falle dass R Alkenyl bedeutet, sind E-konfigurierte Doppelbindungen an C(1) oder C(3) oder aber endständige Doppelbindungen bevorzugt, bei Verbindungen mit aromatischem Ring A solche an C(3) oder endständige. Falls R Alkenyloxy bedeutet, sind die bevorzugten Verbindungen solche mit einer E-konfigurierten Doppelbindung an C(2) oder solche mit einer endständigen Doppelbindung.Gewünschtenfalls kann der Rest R auch1 bis 2 Sauerstoffatome und/oder einen oder mehrere Fluorsubstituenten aufweisen. Dabei sind Reste R mit nur einem Sauerstoffatom sowie solche ohne Fluorsubstituenten bevorzugt. Besonders bevorzugte Reste R sind demnach Methyl, Ethyl, Propyl, Butyl, Pentyl, Hexyl, Methoxy, Ethoxy, Propyloxy, Butyloxy, Pentyloxy, Vinyl, 1-(E)-Propenyl, 1-(E)-Butenyl, 1-(E)-Pentenyl, 1-(E)-Hexenyl, 3-Butenyl, 3-(E)-Pentenyl, 3-(E)-Hexenyl, 4-Pentenyl, Allyloxy, 2-(E)-Butenyloxy, 3-Butenyloxy, Methoxymethyl, Ethoxymethyl, Propyloxymethyl, Allyloxymethyl, Methoxyethyl, Ethoxyethyl, Propyloxyethyl, Methoxypropyl, Ethoxypropyl, Methoxy-1E-propenyl, Ethoxy-1E-propenyl und dergleichen.

In den Verbindungen der allgemeinen Formeln I-1 bis I-8 worin X Alkyl, Alkoxy oder Alkoxyalkyl bedeutet, werden geradkettige Reste mit 1 bis 6 Kohlenstoffatomen bevorzugt, insbesondere werden Reste mit 1 bis 3 Kohlenstoffatomen bevorzugt wie beispielsweise Methyl, Ethyl, Propyl, Methoxy, Ethoxy, Propyloxy, Methoxymethyl, Ethoxymethyl oder Methoxyethyl.

Die gemäss den Formeln I und I-1 bis I-8 definierten Substituenten Y¹ und Y² sind prinzipiell voneinander unabhängig. Bevorzugt sind jedoch diejenigen Verbindungen, bei denen einer der Substituenten Y¹ oder Y² für Wasserstoff und der andere für Fluor oder Wasserstoff steht.

Die Herstellung der Verbindungen der Formel I wird im Schema 1 dargelegt, wobei den in den Formeln I-III angegebenen Symbolen die obenerwähnte Bedeutung zukommt. Die Halogenierung der Verbindungen IIa zu III wird vorzugsweise in einem inerten Lösungsmittel (z.B. Tetrahydrofuran, Ether, Dimethoxyethan) bei tiefer Temperatur (z.B. -70°) durchgeführt. Der Umsatz gelingt auch mit Brom und führt zu den analogen Bromiden, die in der Folgereaktion zu I ebenfalls verwendet werden können.

Metallkatalysierte Kupplungen von Phenylverbindungen mit Acetylenen, wie sie in der letzten Stufe zur Herstellung von I aus III dargestellt wird, sind grundsätzlich aus der Literatur bekannt. Sie können wie bereits erwähnt mit Bromiden und Iodiden, in einigen Fällen aber auch mit Trifluorsulfonaten und Chloriden durchgeführt werden. Ueblicherweise wird dabei das einfach gekuppelte Acetylen isoliert, die Schutzgruppe, hier Isopropyloxy, abgespalten und in einer nächsten Stufe der zweite Phenylring gekuppelt. Wir haben jedoch gefunden, dass der Umsatz von Zwischenprodukten der Formel III zu Verbindungen der Formel I auch in einer Stufe, d.h. ohne dazwischenliegende Aufarbeitung, realisiert werden kann. Dabei wird zunächst eine Verbindung der Formel III mit 2-Methyl-3-butin-2-ol in einem inerten Lösungsmittel wie beispielsweise Tetrahydrofuran mit katalytischen Mengen von Bistriphenylphosphin-palladiumchlorid, Kupfer-I-iodid und Triphenylphosphin unter Anwesenheit von Triethylamin bei erhöhter Temperatur umgesetzt. Danach werden Kaliumhydroxyd und Tetrabutylammoniumhydrogensulfat sowie die Verbindung des Typs IIb zugefügt und bis zum vollständigen Umsatz bei weiterhin erhöhter Temperatur reagieren gelassen. Bei den Verbindungen des Typs IIb können anstelle von Bromiden grundsätzlich auch analoge Iodide, Trifluormethylsulfonate und homologe Polyfluoralkylsulfonate eingesetzt werden.

### Schema 1

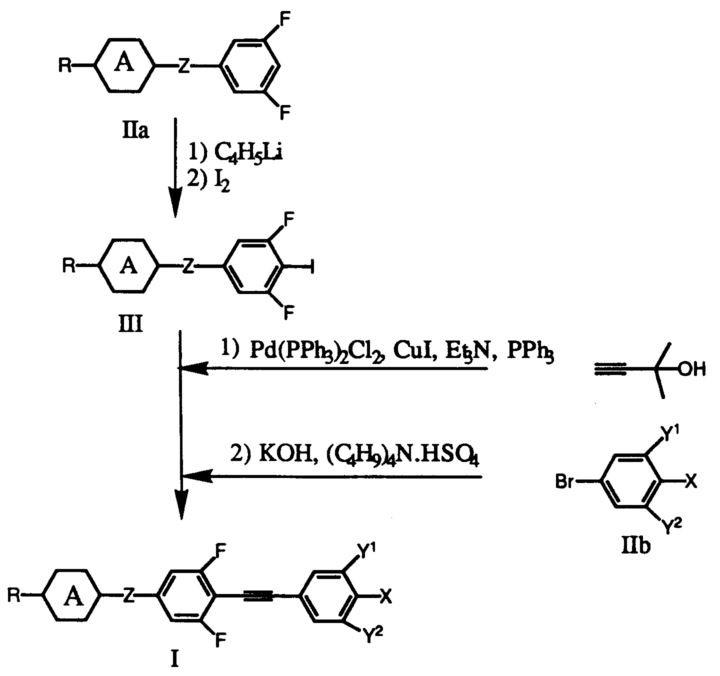

Die Ausgangsmaterialien der Formel IIa sind bekannte oder analoge bekannter Verbindungen und können nach bekannten Methoden hergestellt werden. Solche Verbindungen wurden bereits als Zwischenprodukte für Flüssigkristalle beschrieben. Ihre Synthese erfolgt zudem in völliger Analogie zu den isomeren 3,4-Difluorphenylverbindungen, die dem Fachmann als Flüssigkristalle geläufig sind. Geeignet substituierte Phenylderivate der Formel IIb sind vielfach im Handel erhältlich oder können leicht aus käuflichen Vorstufen nach dem Fachmann bekannten Methoden modifiziert werden.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I und/oder andere Flüssigkristallkomponenten sein.

Die Verbindungen der Formel I eignen sich insbesondere für nematische oder, sofern mindestens eine Komponente des Gemisches optisch aktiv ist, auch für cholesterische Gemische. Ein bevorzugter Anwendungsbereich betrifft die Verwendung als Dielektrikum in Flüssigkristallanzeigevorrichtungen mit verdrillt nematischer Flüssigkristallstruktur, wie TN-Zellen, STN-Zellen, SBE-Zellen, OMI-Zellen und TFT-Zellen. Bevorzugte Gemische sind daher diejenigen, welche eine oder mehrere Verbindungen mit positiver dielektrischer Anisotropie enthalten.

Aufgrund der guten Löslichkeit der Verbindungen der Formel I in anderen Flüssigkristallmaterialien und aufgrund ihrer guten Mischbarkeit untereinander kann der Anteil der Verbindungen der Formel I in den erfindungsgemässen Gemischen relativ hoch sein und beispielsweise etwa 1-70 Gew.-% betragen. Im allgemeinen ist ein Anteil von etwa 3-40 Gew.-%, insbesondere 5-30 Gew.-% an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 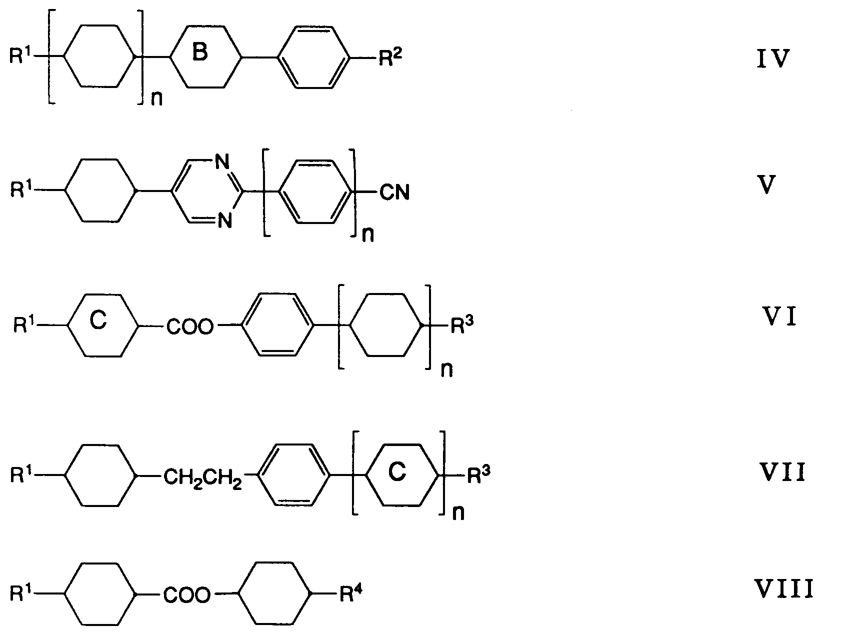 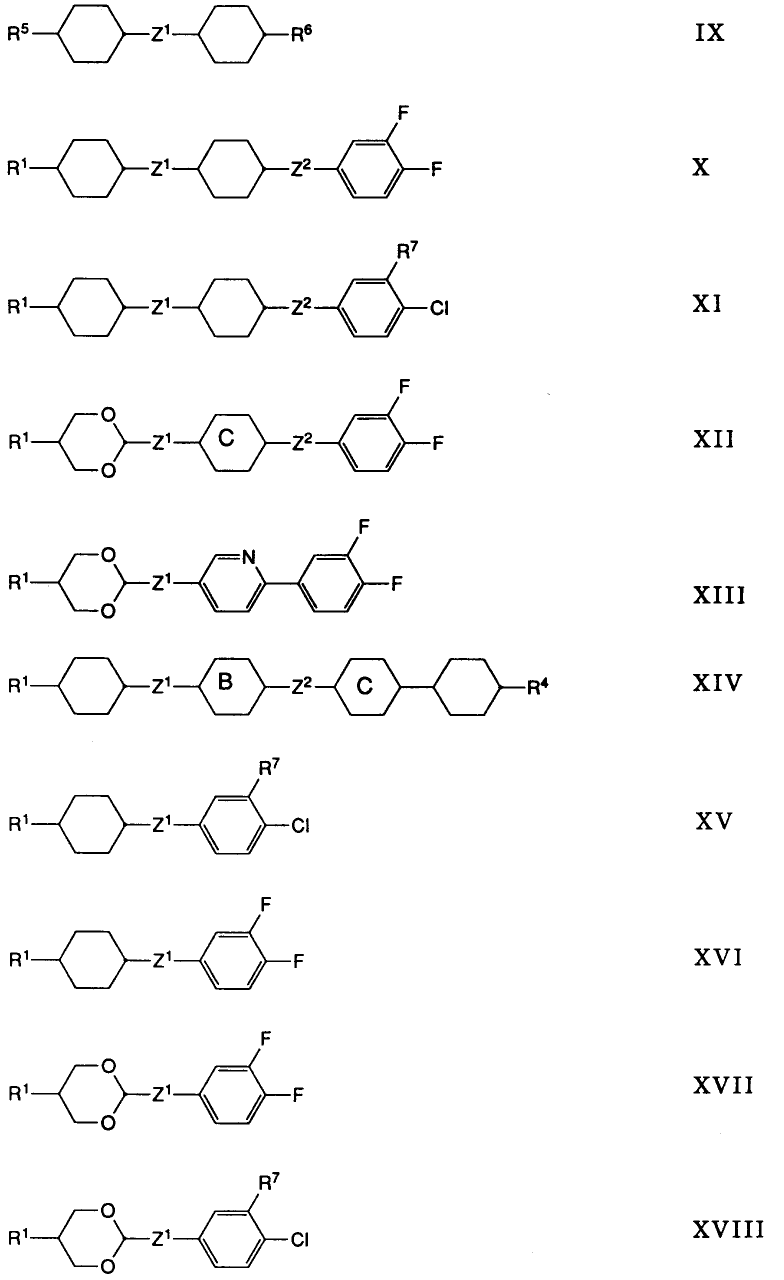 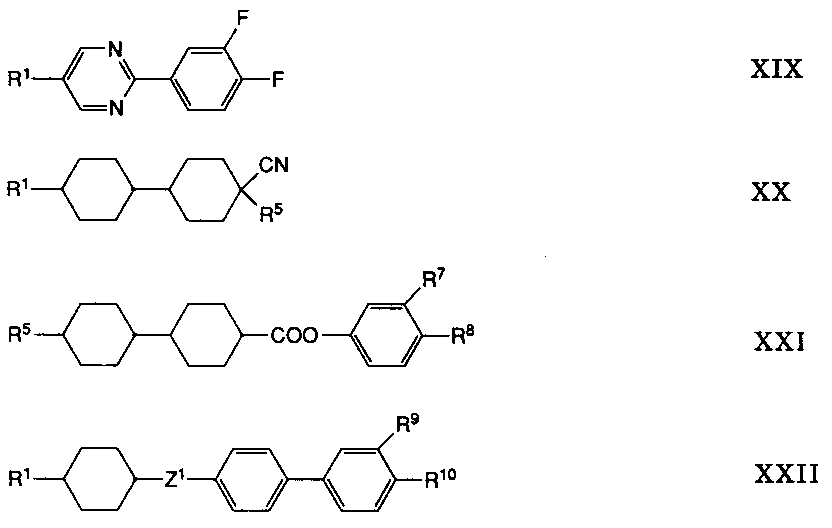 worin
- R¹,R⁴: Alkyl, Alkoxyalkyl, 3E-Alkenyl, 4-Alkenyl oder an gesättigten Ringen auch 1E-Alkenyl bedeuten;
- n: 0 oder 1 bedeutet;
- Ring B: 1,4-Phenylen, Pyridin-2,5-diyl, Pyrimidin-2,5-diyl, trans-1,4-Cyclohexylen oder trans-1,3-Dioxan-2,5-diyl bezeichnet;
- R²: Cyano, Isothiocyanato, Fluor, Alkyl, 3E-Alkenyl, 4-Alkenyl, Alkoxy, 2E-Alkenyloxy, 3-Alkenyloxy oder 1-Alkinyl darstellt;
- Ring C: 1,4-Phenylen oder trans-1,4-Cyclohexylen bedeutet;
- R³: Alkyl, 3E-Alkenyl, 4-Alkenyl, oder an trans-1,4-Cyclohexylen auch 1E-Alkenyl, oder an 1,4-Phenylen auch Cyano, Isothiocyanato, Alkoxy, 2E-Alkenyloxy oder 3-Alkenyloxy bezeichnet;
- R⁵: Alkyl, 1E-Alkenyl, 3E-Alkenyl oder 4-Alkenyl bedeutet;
- R⁶: Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl darstellt;
- Z¹,Z²: unabhängig voneinander eine einfache Kovalenzbindung oder -CH₂CH₂- bezeichnen, wobei zwei aromatische Ringe immer durch eine einfache Kovalenzbindung verknüpft sind;
- R⁷: Wasserstoff, Fluor oder Chlor bedeutet;
- R⁸: Cyano, Fluor oder Chlor darstellt;
- R⁹: Wasserstoff oder Fluor bezeichnet; und
- R¹⁰: Fluor oder Chlor darstellt.

Der obige Ausdruck "gesättigter Ring" umfasst trans-1,4-Cyclohexylen und trans-1,3-Dioxan-2,5-diyl. Die Reste R¹ bis R⁶ besitzen vorzugsweise je 1 bis 12 Kohlenstoffatome, besonders bevorzugt je 1 bis 7 Kohlenstoffatome. Geradkettige Reste sind im allgemeinen bevorzugt.

Der Ausdruck "Alkyl" bedeutet in diesem Zusammenhang vorzugsweise geradkettige Reste mit 1 bis 12 Kohlenstoffatomen, vorzugsweise mit 1 bis 7 Kohlenstoffatomen, wie beispielsweise Methyl, Aethyl, Propvl, Butyl, Pentyl, Hexyl oder Heptyl.

Der Ausdruck "Alkyloxyalkyl" bedeutet in diesem Zusammenhang vorzugsweise geradkettige Reste mit 1 bis 12 Kohlenstoffatomen, besonders bevorzugt mit 1 bis 7 Kohlenstoffatomen, wie beispielsweise Methoxymethyl, Aethoxymethyl, Propyloxymethyl, Butyloxymethyl, Methoxypropyl und dergleichen.

Der Ausdruck "Alkyloxy" bedeutet in diesem Zusammenhang vorzugsweise geradkettige Reste mit 1 bis 12 Kohlenstoffatomen, besonders bevorzugt mit 1 bis 7 Kohlenstoffatomen, wie beispielsweise Methoxy, Aethoxy, Propyloxy, Butyloxy, Pentyloxy, Hexyloxy, Heptyloxy und dergleichen.

Der Ausdruck "1E-Alkenyl" bedeutet in diesem Zusammenhang vorzugsweise geradkettige Alkenylreste mit 2 bis 12, besonders bevorzugt mit 2 bis 7 Kohlenstoffatomen, in denen die Doppelbindung sich in 1-Stellung befindet, wie beispielsweise Vinyl, 1E-Propenyl, 1E-Butenyl, 1E-Pentenyl, 1E-Hexenyl, 1E-Heptenyl und dergleichen.

Der Ausdruck "3E-Alkenyl" bedeutet in diesem Zusammenhang vorzugsweise geradkettige Alkenylreste mit 4 bis 12, besonders bevorzugt mit 4 bis 7 Kohlenstoffatomen, in denen die Doppelbindung sich in 3-Stellung befindet, wie beispielsweise 3-Butenyl, 3E-Pentenyl, 3E-Hexenyl, 3E-Heptenyl und dergleichen.

Der Ausdruck "4-Alkenyl" bedeutet in diesem Zusammenhang vorzugsweise geradkettige Alkenylreste mit 5 bis 12 Kohlenstoffatomen, in denen die Doppelbindung sich in 4-Stellung befindet, wie beispielsweise 4-Pentenyl, 4-Hexenyl, 4-Heptenyl und dergleichen.

Der Ausdruck "2E- bzw. 3Z-Alkenyloxy" bedeutet in diesem Zusammenhang vorzugsweise geradkettige Alkenyloxyreste mit 3 bzw. 4 bis 12 Kohlenstoffatomen, besonders bevorzugt mit 3 bzw. 4 bis 7 Kohlenstoffatomen, in denen die Doppelbindung sich in 2- bzw. 3-Stellung befindet und E bzw. Z die bevorzugte Konfiguration angibt, wie beispielsweise Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy und dergleichen.

Der Ausdruck "1-Alkinyl" bedeutet in diesem Zusammenhang vorzugsweise geradkettige Alkinylreste mit 2 bis 12, besonders bevorzugt mit 2 bis 7 Kohlenstoffatomen, in denen die Dreifachbindung sich in 1-Stellung befindet, wie beispielsweise Aethinyl, 1-Propinyl, 1-Butinyl, 1-Pentinyl und dergleichen.

Die Herstellung der flüssigkristallinen Gemische und der elektrooptischen Vorrichtungen kann in an sich bekannter Weise erfolgen.

Die Erfindung wird durch die folgenden Beispiele weiter veranschaulicht. In den Beispielen bedeutet C eine kristalline, N eine nematische, S eine smektische und I die isotrope Phase. V₁₀ bezeichnet die Spannung für 10% Transmission. tₒₙ und t_{off} bezeichnen die Einschaltzeit bzw. die Ausschaltzeit. Δn bezeichnet die optische Anisotropie.

### Beispiel 1

a) Zu einer Lösung von 2,14 g 1-(trans-4-Propylcyclohexyl)-3,5,-difluorbenzol in 20 ml trockenem Tetrahydrofuran bei -70°C wurden 5,9 ml einer 1,6 N Butyllithiumlösung in Hexan während 20 Min. getropft und während 1 Std. bei -70°C reagieren gelassen. Dann wurde bei -60° eine Lösung von 2,4 g Iod in 10 ml trockenem Tetrahydrofuran innert 10 Min. zugetropft und während weiteren 30 Min. allmählich auf Raumtemperatur erwärmt. Die entstandene gelbe Lösung wurde mit 10 ml Wasser und dann mit 10 ml einer 10proz. wässrigen Natrium-bicarbonatlösung versetzt und mit Ether extrahiert. Die Etherlösung wurde mit ges. Kochsalzlösung und mehrmals mit Wasser gewaschen, über Magnesiumsalfat getrocknet, filtriert und eingeengt. Chromatographie des Rückstandes über 150 g Kieselgel mit Hexan ergab 3,27 g 1-(trans-4-Propylcyclohexyl)-3,5,-difluor-4-iodbenzol als farblose Flüssigkeit.
b) Ein Gemisch von 1 g 1-(trans-4-Propylcyclohexyl)-3,5,-difluor-4-iodbenzol, 0.294 ml 2-Methyl-3-butin-2-ol, 0.077 g Bis(triphenylphosphin)palladiumdichlorid, 0.842 g Triethylamin, 0.084 g Triphenylphosphin und 10 ml Tetrahydrofuran wurden während 48 Std. unter Rückfluss gehalten. Dann wurden 0.326 ml 1-Brom-3,4-difluorbenzol, 0.493 g gepulvertes Kaliumhydroxyd und 0.205 g Tetrabutylammoniumhydrogensulfat in dieser Reihenfolge zugegeben und während 16 Std. unter Rückfluss reagieren gelassen. Hierauf wurde das abgekühlte Reaktions-gemisch auf 25 ml 1N Schwefelsäure gegossen und mit Ether extrahiert. Die Etherphase wurde mehrmals mit ges. Kochsalzlösung gewaschen, über Magnesiumsalfat getrocknet und am Rotationsverdampfer eingedampft. Chromatographie des Rückstandes an 170 g Kieselgel mit Hexan und anschliessende Kristallisation aus Methanol ergab 0.53 g 4-(trans-4-Propylcyclohexyl)-2,6,3',4'-tetrafluortolan. Smp. (C/N) 71.3°C, Klp. (N/I) 126°C.

Auf analoge Weise können die folgenden Verbindungen hergestellt werden:
4-(trans-4-Ethylcyclohexyl)-2,6,3',4'-tetrafluortolan;
4-(trans-4-Butylcyclohexyl)-2,6,3',4'-tetrafluortolan;
4-(trans-4-Pentylcyclohexyl)-2,6,3',4'-tetrafluortolan;
4-(trans-4-Vinylcyclohexyl)-2,6,3',4'-tetrafluortolan;
4-[trans-4-(1E-Propenyl)cyclohexyl]-2,6,3',4'-tetrafluortolan;
4-[trans-4-(3-Butenyl)cyclohexyl]-2,6,3',4'-tetrafluortolan;
4-[trans-4-(3- Methoxy-1E-propenyl)cyclohexyl]-2,6,3',4'-tetrafluortolan;
4-[trans-4-(3-Methoxypropyl)cyclohexyl]-2,6,3',4'-tetrafluortolan;
4-(trans-4-Propylcyclohexyl)-2,6,4'-trifluortolan;
4-(trans-4-Propylcyclohexyl)-2,6-difluor-4'-chlortolan, Smp. (C/N) 100,4, Klp. (N/I) 188,8°C;
4-(trans-4-Vinylcyclohexyl)-2,6-difluor-4'-chlortolan;
4-[trans-4-(3-Methoxypropyl)cyclohexyl]-2,6-difluor-4'-chlortolan;
4-(trans-4-Propylcyclohexyl)-2,6,3'-trifluor-4'-chlortolan; Smp (C/N) 90°, Klp. (N/I) 158,9°;
4-[trans-4-(1E-Propenyl)cyclohexyl]-2,6,3'-trifluor-4'-chlortolan;
4-[trans-4-(3-Methoxypropyl)cyclohexyl]-2,6,3'-trifluor-4'-chlortolan;
4-(trans-4-Propylcyclohexyl)-2,6,3'-trifluor-4'-cyanotolan, Smp. (C/N) 134,8°C, Klp. (N/I) 211,8°C;
4-(trans-4-Propylcyclohexyl)-2,6,3'-trifluor-4'-trifluormethyltolan;
4-(trans-4-Propylcyclohexyl)-2,6-difluor-4'-trifluormethoxytolan, Smp. (C/S) 56°C, (S/N) 84.5°C, Klp. (N/I) 169.4°C;
4-(trans-4-Propylcyclohexyl)-2,6,3'-trifluor-4'-trifluormethoxytolan;
4-(trans-4-Propylcyclohexyl)-2,6-difluor-4'-difluormethoxytolan, Smp. (C/N) 80,5°C, Klp. (N/I) 181,2°C;
4-(trans-4-Propylcyclohexyl)-2,6,3'-trifluor-4'-difluormethoxytolan, Smp. (C/N) 47.5°C, Klp (N/I) 155.6°C;
4-(trans-5-Propyl-1,3-dioxan-2-yl)-2,6,3',4'-tetrafluortolan;
4-(trans-5-Butyl-1,3-dioxan-2-yl)-2,6,3',4'-tetrafluortolan;
4-(trans-5-Pentyl-1,3-dioxan-2-yl)-2,6,3',4'-tetrafluortolan;
4-[trans-5-(1E-Propenyl)-1,3-dioxanyl]-2,6,3',4'-tetrafluortolan, Smp. (C/N) 99,4°C, Klp. (N/I) 151,9°C;
4-[trans-5-(3-Butenyl)-1,3-dioxanyl]-2,6,3',4'-tetrafluortolan;
4-[trans-5-(3-Methoxy-1E-propenyl)-1,3-dioxan-2-yl]-2,6,3',4'-tetrafluortolan;
4-[trans-5-(3-Methoxypropyl)-1,3-dioxan-2-yl]-2,6,3',4'-tetrafluortolan;
4-(trans-5-Propyl-1,3-dioxan-2-yl)-2,6,4'-trifluortolan;
4-(trans-5-Propyl-1,3-dioxan-2-yl)-2,6-difluor-4'-chlortolan;
4-[trans-5-(3-Methoxypropyl)-1,3-dioxanyl]-2,6-difluor-4'-chlortolan;
4-(trans-5-Propyl-1,3-dioxan-2-yl)-2,6,3'-trifluor-4'-chlortolan;
4-[trans-5-(1E-Propenyl)-1,3-dioxanyl]-2,6,3'-trifluor-4'-chlortolan, Smp. (C/N) 117°C, Klp. (N/I) 184,4°C;
4-[trans-5-(3-Methoxypropyl)-1,3-dioxan-2-yl]-2,6,3'-trifluor-4'-chlortolan;
4-(trans-5-Propyl-1,3-dioxan-2-yl)-2,6,3'-trifluor-4'-cyanotolan;
4-[trans-5-(1E-Propenyl)-1,3-dioxan-2-yl]-2,6,3'-trifluor-4'-cyanotolan, Smp (C/N) 156°C, Klp. (N/I) >225°C.
4-(trans-5-Propyl-1,3-dioxan-2-yl)-2,6-difluor-4'-ethyltolan;
4-(trans-5-Propyl-1,3-dioxan-2-yl)-2,6-difluor-4'-ethoxytolan;
4-(trans-5-Propyl-1,3-dioxan-2-yl)-2,6,3'-trifluor-4'-methyltolan;
4-(trans-5-Propyl-1,3-dioxan-2-yl)-2,6,3'-trifluor-4'-methoxytolan;
4-(trans-5-Propyl-1,3-dioxan-2-yl)-2,6,3'-trifluor-4'-methoxymethyltolan;
4-(trans-5-Propyl-1,3-dioxan-2-yl)-2,6,3'-trifluor-4'-trifluormethyltolan;
4-(trans-5-Propyl-1,3-dioxan-2-yl)-2,6,3'-trifluor-4'-trifluormethoxytolan;
4-(trans-5-Propyl-1,3-dioxan-2-yl)-2,6,3'-trifluor-4'-difluormethoxytolan;
4-[2-(trans-4-Propylcyclohexyl)ethyl]-2,6,3',4'-tetrafluortolan;
4-[2-(trans-4-Butylcyclohexyl)ethyl]-2,6,3',4'-tetrafluortolan;
4-[2-(trans-4-Pentylcyclohexyl)ethyl]-2,6,3',4'-tetrafluortolan;
4-[2-(trans-4-Vinylcyclohexyl)ethyl]-2,6,3',4'-tetrafluortolan;
4-[2-[trans-4-(1E-Propenyl)cyclohexyl]ethyl]-2,6,3',4'-tetrafluortolan;
4-[2-[trans-4-(3-Butenyl)cyclohexyl]ethyl]-2,6,3',4'-tetrafluortolan;
4-[2-[trans-4-(3- Methoxy-1E-propenyl)ethyl]cyclohexyl]-2,6,3',4'-tetrafluortolan;
4-[2-[trans-4-(3-Methoxypropyl)cyclohexyl]ethyl]-2,6,3',4'-tetrafluortolan;
4-[2-(trans-4-Propylcyclohexyl)ethyl]-2,6,4'-trifluortolan;
4-[2-(trans-4-Propylcyclohexyl)ethyl]-2,6-difluor-4'-chlortolan;
4-[2-(trans-4-Vinylcyclohexyl)ethyl]-2,6-difluor-4'-chlortolan;
4-[2-[trans-4-(3-Methoxypropyl)cyclohexyl]ethyl]-2,6-difluor-4'-chlortolan;
4-[2-(trans-4-Propylcyclohexyl)ethyl]-2,6,3'-trifluor-4'-chlortolan;
4-[2-[trans-4-(1E-Propenyl)cyclohexyl]ethyl]-2,6,3'-trifluor-4'-chlortolan;
4-[2-[trans-4-(3-Methoxypropyl)cyclohexyl]ethyl]-2,6,3'-trifluor-4'-chlortolan;
4-[2-(trans-4-Propylcyclohexyl)ethyl]-2,6,3'-trilluor-4'-cyanotolan;
4-[2-(trans-4-Propylcyclohexyl)ethyl]-2,6,3'-trifluor-4'-trifluormethyltolan;
4-[2-(trans-4-Propylcyclohexyl)ethyl-2,6,3'-trifluor-4'-trifluormethoxytolan;
4-[2-(trans-4-Propylcyclohexyl)ethyl]-2,6,3'-trifluor-4'-difluormethoxytolan;
4-[(trans-4-Propylcyclohexyl)methoxy]-2,6,3',4'-tetrafluortolan;
4-[(trans-4-Propylcyclohexyl)methoxy]-2,6,3'-trifluor-4'-chlortolan;
4-[2-(trans-5-Propyl-1,3-dioxan-2-yl)ethyl]-2,6,3',4'-tetrafluortolan;
4-[2-[trans-5-(1E-Propenyl)-1,3-dioxan-2-yl)ethyl]-2,6,3',4'-tetrafluortolan;
4-[2-(trans-5-(3-Methoxypropyl)-1,3-dioxan-2-yl)ethyl]-2,6,3',4'-tetrafluortolan;
4-[2-(trans-5-Propyl-1,3-dioxan-2-yl)ethyl]-2,6,3'-trifluor-4'-chlortolan;
4-(4-Ethylphenyl)-2,6,3',4'-tetrafluortolan;
4-(4-Propylphenyl)-2,6,3',4'-tetrafluortolan, Smp. (C/I) 90°C;
4-(4-Butylphenyl)-2,6,3',4'-tetrafluortolan;
4-(4-Pentylphenyl)-2,6,3',4'-tetrafluortolan;
4-(4-Hexylphenyl)-2,6,3',4'-tetrafluortolan;
4-(4-Heptylphenyl)-2,6,3',4'-tetrafluortolan;
4-[4-(3-Butenyl)phenyl]-2,6,3',4'-tetrafluortolan;
4-[4-(3E-Pentenyl)phenyl]-2,6,3',4'-tetrafluortolan;
4-(4-Ethoxyphenyl)-2,6,3',4'-tetrafluortolan;
4-(4-Propyloxyphenyl)-2,6,3',4'-tetrafluortolan;
4-(4-Butyloxyphenyl)-2,6,3',4'-tetrafluortolan;
4-(4-Pentyloxyhenyl)-2,6,3',4'-tetrafluortolan;
4-(4-Allyloxyhenyl)-2,6,3',4'-tetrafluortolan;
4-[4-(3-Methoxypropyl)phenyl]-2,6,3',4'-tetrafluortolan;
4-(4-Propylphenyl)-2,6,3'-trifluor-4'-chlortolan, Smp. (C/N) 92,3°C, Klp. (N/I) 142,7°C;
4-(4-Propyloxyphenyl)-2,6,3'-trifluor-4'-chlortolan;
4-(4-Propylphenyl)-2,6,3'-trifluor-4'-cyanotolan;
4-(4-Propylphenyl)-2,6,3'-trifluor-4'-trifluormethyltolan;
4-(4-Propylphenyl)-2,6,3'-trifluor-4'-trifluormethoxytolan;
4-(4-Propylphenyl)-2,6,3'-trifluor-4'-difluormethoxytolan;
4-(5-Ethylppyridin-2-yl)-2,6,3',4'-tetrafluortolan;
4-(5-Propylpyridin-2-yl)-2,6,3',4'-tetrafluortolan, Smp. (C/N) 101,1°C, Klp. (N/I) 131,7°C;
4-(5-Butylpyridin-2-yl)-2,6,3',4'-tetrafluortolan;
4-(5-Pentylpyridin-2-yl)-2,6,3',4'-tetrafluortolan;
4-(5-Ethoxypyridin-2-yl)-2,6,3',4'-tetrafluortolan;
4-(5-Propyloxypyridin-2-yl)-2,6,3',4'-tetrafluortolan;
4-(5-Allyloxypyridin-2-yl)-2,6,3',4'-tetrafluortolan;
4-[5-(3-Methoxypropyl)pyridin-2-yl]-2,6,3',4'-tetrafluortolan;
4-(5-Propylpyridin-2-yl)-2,6,3'-trifluor-4'-chlortolan, Smp. (C/N) 113,4°C, Klp. (N/I) 161,7°C;
4-(5-Butylpyridin-2-yl)-2,6,3'-trifluor-4'-chlortolan;
4-(5-Pentylpyridin-2-yl)-2,6,3'-trifluor-4'-chlortolan;
4-(5-(3-Methoxypropyl)pyridin-2-yl)-2,6,3'-trifluor-4'-chlortolan;
4-(5-Ethylpyrimidin-2-yl)-2,6,3',4'-tetrafluortolan;
4-(5-Propylpyrimidin-2-yl)-2,6,3',4'-tetrafluortolan, Smp. (C/N) 144,6°C, Klp. (N/I) 154°C;
4-(5-Butylpyrimidin-2-yl)-2,6,3',4'-tetrafluortolan;
4-(5-Pentylpyrimidin-2-yl)-2,6,3',4'-tetrafluortolan;
4-(5-Ethoxyprimidin-2-yl)-2,6,3',4'-tetrafluortolan;
4-(5-Propyloxypyrimidin-2-yl)-2,6,3',4'-tetrafluortolan;
4-(5-Allyloxypyrimidin-2-yl)-2,6,3',4'-tetrafluortolan;
4-[5-(3-Methoxypropyl)pyrimidin-2-yl]-2,6,3',4'-tetrafluortolan;
4-(5-Propylpyrimidin-2-yl)-2,6,3'-trifluor-4'-chlortolan, Smp. (C/N) 160,3°C, Klp. (N/I) 176,5°C;
4-(5-Butylpyrimidin-2-yl)-2,6,3'-trifluor-4'-chlortolan;
4-(5-Pentylpyrimidin-2-yl)-2,6,3'-trifluor-4'-chlortolan;
4-(5-Allyloxypyrimidin-2-yl)-2,6,3'-trifluor-4'-chlortolan;
4-(5-(3-Methoxypropyl)pyrimidin-2-yl)-2,6,3'-trifluor-4'-chlortolan.

### Beispiel 2

Zur Untersuchung der Eigenschaften der Verbindungen der Formel I in Gemischen wurden binäre Mischungen (BM) mit 4-(trans-4-Pentylcyclohexyl)benzonitril hergestellt. Die Schwellenspannung und die Ansprechzeiten wurden in einer TN-Zelle (low bias tilt) mit 8 mm Plattenabstand bei 22°C gemessen; als Betriebsspannung wurde der 2,5-fache Wert der Schwellenspannung (V₁₀) gewählt. Die entsprechenden Daten für 4-(trans-4-Pentylcyclohexyl)benzonitril betragen: Klp. (N/I) = 54.6°C, V₁₀ = 1,62 V, tₒₙ = 22ms, t_{off} = 42ms, Δn = 0,120.

### BM-1

- 90 Gew.%: 4-(trans-4-Pentylcyclohexyl)benzonitril
- 10 Gew.%: 4-(trans-4-Propylcyclohexyl)-2,6,3',4'-tetrafluortolan
- Klp. (N/I):: 57.3°C, V₁₀ = 1,57 V, tₒₙ = 26ms, t_{off} = 43ms, Δn = 0,129

### BM-2

- 80 Gew.%: 4-(trans-4-Pentylcyclohexyl)benzonitril
- 20 Gew.%: 4-(trans-4-Propylcyclohexyl)-2,6,3',4'-tetrafluortolan
- Klp. (N/I):: 60.3°C, V₁₀ = 1,51 V, tₒₙ = 28 ms, t_{off} = 47 ms, Δn = 0,137

### BM-3

- 90 Gew.%: 4-(trans-4-Pentyl-cyclohexyl)benzonitril
- 10 Gew.%: 4-[trans-5-(1E-Propenyl)-1,3-dioxan-2-yl]-2,6,3',4'-tetrafluortolan;
- Klp. (N/I):: 56.5°C, V₁₀ = 1,50 V, tₒₙ = 26 ms, t_{off} = 43 ms, Δn = 0,132

### BM-4

- 80 Gew.%: 4-(trans-4-Pentyl-cyclohexyl)benzonitril
- 20 Gew.%: 4-[trans-5-(1E-Propenyl)-1,3-dioxan-2-yl]-2,6,3',4'-tetrafluortolan;
- Klp. (N/I):: 59.9°C, V₁₀ = 1,42 V, tₒₙ = 29 ms, t_{off} = 49 ms, Δn = 0,140

### BM-5

- 90 Gew.%: 4-(trans-4-Pentyl-cyclohexyl)benzonitril
- 10 Gew.%: 4-[trans-5-(1E-Propenyl)-1,3-dioxan-2-yl]-2,6,3'-trifluor-4'-chlortolan;
- Klp. (N/I):: 58.3°C, V₁₀ = 1,50 V, tₒₙ = 28 ms, t_{off} = 43 ms, Δn = 0,136.

### BM-6

- 80 Gew.%: 4-(trans-4-Pentylcyclohexyl)benzonitril
- 20 Gew.%: 4-[trans-5-(1E-Propenyl)-1,3-dioxan-2-yl]-2,6,3'-trifluor-4'-chlortolan;
- Klp. (N/I):: 63.9°C, V₁₀ = 1,41 V, tₒₙ = 31 ms, t_{off} = 47 ms.

### BM-7

- 90 Gew.%: 4-(trans-4-Pentyl-cyclohexyl)benzonitril
- 10 Gew.%: 4-(4-Propylphenyl)-2,6,3',4'-tetrafluortolan
- Klp. (N/I):: 54.0°C, V₁₀ = 1,52 V, tₒₙ = 29 ms, t_{off} = 44 ms, Δn = 0,138

### BM-8

- 80 Gew.%: 4-(trans-4-Pentyl-cyclohexyl)benzonitril
- 20 Gew.%: 4-(4-Propylphenyl)-2,6,3',4'-tetrafluortolan
- Klp. (N/I):: 53.1°C, V₁₀ = 1,46 V, tₒₙ = 27 ms, t_{off} = 45 ms, Δn = 0,153.

### BM-9

- 90 Gew.%: 4-(trans-4-Pentyl-cyclohexyl)benzonitril
- 10 Gew.%: 4-(4-Propylphenyl)-2,6,3'-trifluor-4'-chlortolan
- Klp. (N/I):: 58.1°C, V₁₀ = 1,61 V, tₒₙ = 27 ms, t_{off} = 42 ms, Δn = 0,143.

### BM-10

- 80 Gew.%: 4-(trans-4-Pentyl-cyclohexyl)benzonitril
- 20 Gew.%: 4-(4-Propylphenyl)-2,6,3'-trifluor-4'-chlortolan
- Klp. (N/I):: 62.3°C, V₁₀ = 1,55 V, tₒₙ = 30 ms, t_{off} = 47 ms, Δn = 0,164.

### BM-11

- 90 Gew.%: 4-(trans-4-Pentyl-cyclohexyl)benzonitril
- 10 Gew.%: 4-(5-Propylpyridin-2-yl)-2,6,3',4'-tetrafluortolan
- Klp. (N/I):: 56.1°C, V₁₀ = 1,40 V, tₒₙ = 28 ms, t_{off} = 45 ms, Δn = 0,138.

### BM-12

- 80 Gew.%: 4-(trans-4-Pentyl-cyclohexyl)benzonitril
- 20 Gew.%: 4-(5-Propylpyridin-2-yl)-2,6,3',4'-tetrafluortolan
- Klp. (N/I):: 58.3°C, V₁₀ = 1,29 V, tₒₙ = 35 ms, t_{off} = 51 ms, Δn = 0,155.

### BM-13

- 90 Gew.%: 4-(trans-4-Pentyl-cyclohexyl)benzonitril
- 10 Gew.%: 4-(5-Propylpyridin-2-yl)-2,6,3'-trifluor-4'-chlortolan
- Klp. (N/I):: 57.8°C, V₁₀ = 1,49 V, tₒₙ = 27 ms, t_{off} = 45 ms, Δn = 0,146.

### BM-14

- 80 Gew.%: 4-(trans-4-Pentyl-cyclohexyl)benzonitril
- 20 Gew.%: 4-(5-Propylpyridin-2-yl)-2,6,3'-trifluor-4'-chlortolan
- Klp. (N/I):: 63.0°C, V₁₀ = 1,39 V, tₒₙ = 32 ms, t_{off} = 52 ms, Δn = 0,162.

### BM-15

- 90 Gew.%: 4-(trans-4-Pentyl-cyclohexyl)benzonitril
- 10 Gew.%: 4-(5-Propylpyrimidin-2-yl)-2,6,3',4'-tetrafluortolan
- Klp. (N/I):: 56.8°C, V₁₀ = 1,42 V, tₒₙ = 26 ms, t_{off} = 43 ms, Δn = 0,126.

### BM-16

- 80 Gew.%: 4-(trans-4-Pentyl-cyclohexyl)benzonitril
- 20 Gew.%: 4-(5-Propylpyrimidin-2-yl)-2,6,3',4'-tetrafluortolan
- Klp. (N/I):: 57.6°C, V₁₀ = 1,39 V, tₒₙ = 27 ms, t_{off} = 47 ms.

### BM-17

- 90 Gew.%: 4-(trans-4-Pentyl-cyclohexyl)benzonitril
- 10 Gew.%: 4-(trans-4-Propylcyclohexyl)-2,6-difluor-4'-trifluormethoxytolan
- Klp. (N/I):: 58.7°C, V₁₀ = 1,53 V, tₒₙ = 27 ms, t_{off} = 44 ms, Δn = 0,130.

### BM-18

- 80 Gew.%: 4-(trans-4-Pentyl-cyclohexyl)benzonitril
- 20 Gew.%: 4-(trans-4-Propylcyclohexyl)-2,6-difluor-4'-trifluormethoxytolan
- Klp. (N/I):: 64.3°C, V₁₀ = 1,58 V, tₒₙ = 27 ms, t_{off} = 46 ms, Δn = 0.140.

### BM-19

- 90 Gew.%: 4-(trans-4-Pentyl-cyclohexyl)benzonitril
- 10 Gew.%: 4-(trans-4-Propylcyclohexyl)-2,6-difluor-4'-difluormethoxytolan
- Klp. (N/I):: 60.4°C, V₁₀ = 1,52 V, tₒₙ = 25 ms, t_{off} = 39 ms, Δn = 0,133.

### BM-20

- 80 Gew.%: 4-(trans-4-Pentyl-cyclohexyl)benzonitril
- 20 Gew.%: 4-(trans-4-Propylcyclohexyl)-2,6-difluor-4'-difluormethoxytolan
- Klp. (N/I):: 67.4°C, V₁₀ = 1,48 V, tₒₙ = 32 ms, t_{off} = 47 ms, Δn = 0,144.

### BM-21

- 90 Gew.%: 4-(trans-4-Pentyl-cyclohexyl)benzonitril
- 10 Gew.%: 4-(trans-4-Propylcyclohexyl)-2,6,3'-trifluor-4'-difluormethoxytolan
- Klp. (N/I):: 58.0°C, V₁₀ = 1,43 V, tₒₙ = 30 ms, t_{off} = 44 ms, Δn = 0,130.

### BM-22

- 80 Gew.%: 4-(trans-4-Pentyl-cyclohexyl)benzonitril
- 20 Gew.%: 4-(trans-4-Propylcyclohexyl)-2,6,3'-trifluor-4'-difluormethoxytolan
- Klp. (N/I):: 62.4°C, V₁₀ = 1,41 V, tₒₙ = 34 ms, t_{off} = 51 ms, Δn = 0,139.

## Patentansprüche (Patentansprüche für folgende(n) Vertragsstaat(en): CH, FR, GB, IT, LI, NL)

1. Verbindungen der allgemeinen Formel 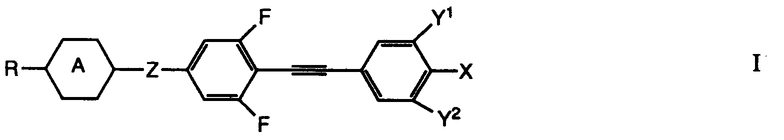 worin
Ring A 1,4-Phenylen, Pyridin-2,5-diyl, Pyrimidin-2,5-diyl, trans-1,4-Cyclohexylen oder trans 1,3-Dioxan-2,5-diyl darstellt;
Z eine einfache Kovalenzbindung, -CH₂CH₂-, -OCH₂-, -CH₂O-, -(CH₂)₄-, -O(CH₂)₃-, -(CH₂)₃O-, oder, falls Ring A einen gesättigten Ring darstellt, auch die trans-Form von -CH=CH(CH₂)₂- oder -CH=CHCH₂O- bedeutet;
X Fluor, Chlor, Cyano, -CF₃, -OCF₃, -OCHF₂, Alkyl, Alkoxy oder Alkoxyalkyl mit 1 bis 6 Kohlenstoffatomen bedeutet;
Y¹, Y² unabhängig voneinander Fluor oder Wasserstoff bedeuten ; und
R Alkyl, Alkoxy, Alkenyl oder Alkenyloxy mit 1 bzw. 2 bis 12 Kohlenstoffatomen bedeutet, in welchen eine oder zwei miteinander nicht benachbarte CH₂-Gruppen durch Sauerstoff und/oder eines oder mehrere Wasserstoffatome durch Fluoratome ersetzt sein können.

2. Verbindungen der allgemeinen Formel I gemäss Anspruch 1, dadurch gekennzeichnet, dass Z eine einfache Kovalenzbindung, -CH₂CH₂- oder -CH₂O- bedeutet.

3. Verbindungen gemäss einem der Ansprüche 1 und 2 der allgemeinen Formeln 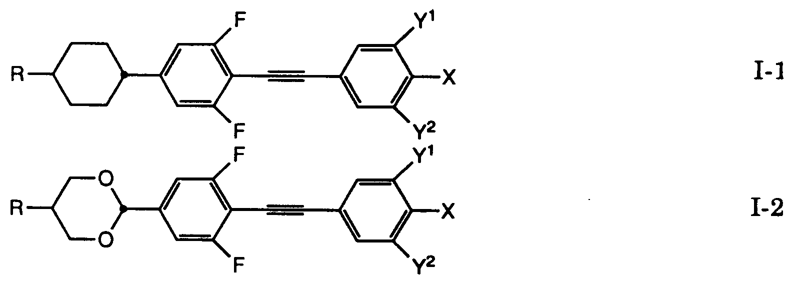 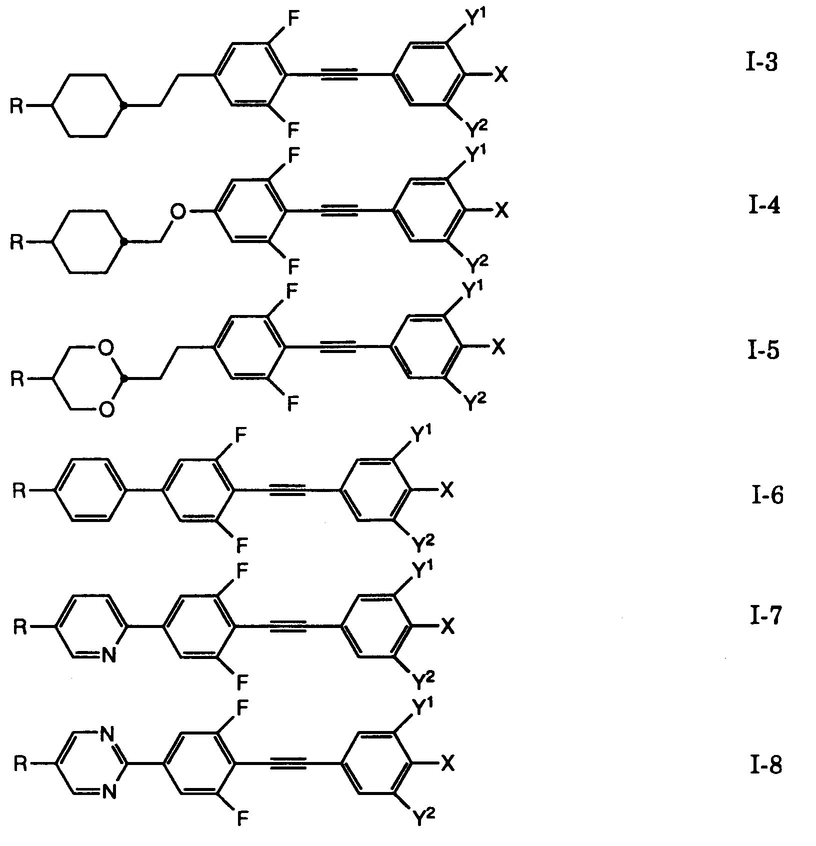 worin
X Fluor, Chlor, Cyano, -CF₃, -OCF₃, -OCHF₂, Alkyl, Alkoxy oder Alkoxyalkyl mit 1 bis 6 Kohlenstoffatomen bedeutet;
Y¹, Y² unabhängig voneinander Fluor oder Wasserstoff bedeuten ; und
R Alkyl, Alkoxy, Alkenyl oder Alkenyloxy mit 1 bzw. 2 bis 12 Kohlenstoffatomen bedeutet, in welchen eine CH₂-Gruppe durch Sauerstoff ersetzt sein kann und/oder eines oder mehrere Wasserstoffatome durch Fluoratome ersetzt sein können.

4. Verbindungen gemäss einem der Ansprüche 1 bis 3, dadurch gekennzeichnet, dass R Alkyl, Alkoxy, Alkenyl oder Alkenyloxy, mit 1 bzw. 2 bis 6 Kohlenstoffatomen, bedeutet, in welchen eine CH₂-Gruppe durch Sauerstoff ersetzt sein kann.

5. Verbindungen gemäss einem der Ansprüche 1 bis 4, dadurch gekennzeichnet, dass einer der Substituenten Y¹ oder Y² Wasserstoff und der andere Wasserstoff oder Fluor bedeutet.

6. Flüssigkristalline Gemische enthaltend mindestens zwei Komponenten, dadurch gekennzeichnet, dass mindestens eine Komponente eine Verbindung der in Anspruch 1 definierten Formel I ist.

7. Flüssigkristalline Gemische gemäss Anspruch 6, dadurch gekennzeichnet, dass der Anteil an Verbindungen der in Anspruch 1 definierten Formel I 3-40 Gew.-% beträgt.

8. Flüssigkristalline Gemische gemäss einem der Ansprüche 6 oder 7, dadurch gekennzeichnet, dass der Anteil an Verbindungen der in Anspruch 1 definierten Formel I 5-30 Gew.-% beträgt.

9. Verwendung der in Anspruch 1 definierten Verbindungen der Formel I für elektro-optische Zwecke.

## Patentansprüche (Patentansprüche für folgende(n) Vertragsstaat(en): DE)

1. Verbindungen der allgemeinen Formel 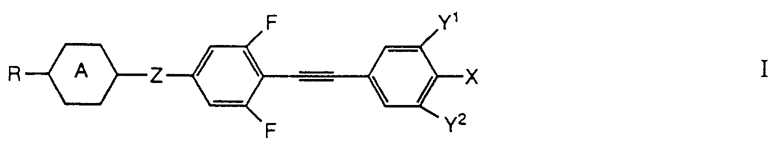 worin
Ring A Pyridin-2,5-diyl, Pyrimidin-2,5-diyl, oder trans 1,3-Dioxan-2,5-diyl darstellt;
Z eine einfache Kovalenzbindung, -CH₂CH₂-, -OCH₂-, -CH₂O-, -(CH₂)₄-, -O(CH₂)₃-, -(CH₂)₃O-, oder, falls Ring A einen gesättigten Ring darstellt, auch die trans-Form von -CH=CH(CH₂)₂- oder -CH=CHCH₂O- bedeutet;
X Fluor, Chlor, Cyano, -CF₃, -OCF₃, -OCHF₂, Alkyl, Alkoxy oder Alkoxyalkyl mit 1 bis 6 Kohlenstoffatomen bedeutet;
Y¹, Y² unabhängig voneinander Fluor oder Wasserstoff bedeuten ; und
R Alkyl, Alkoxy, Alkenyl oder Alkenyloxy mit 1 bzw. 2 bis 12 Kohlenstoffatomen bedeutet, in welchen eine oder zwei miteinander nicht benachbarte CH₂-Gruppen durch Sauerstoff und/oder eines oder mehrere Wasserstoffatome durch Fluoratome ersetzt sein können.

2. Verbindungen der allgemeinen Formel I gemäss Anspruch 1, dadurch gekennzeichnet, dass Z eine einfache Kovalenzbindung, -CH₂CH₂- oder -CH₂O- bedeutet.

3. Verbindungen gemäss einem der Ansprüche 1 und 2 der allgemeinen Formeln 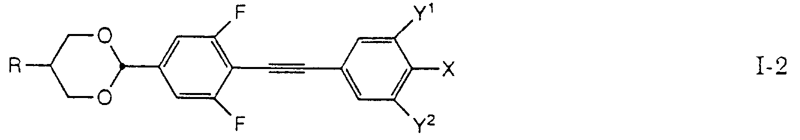 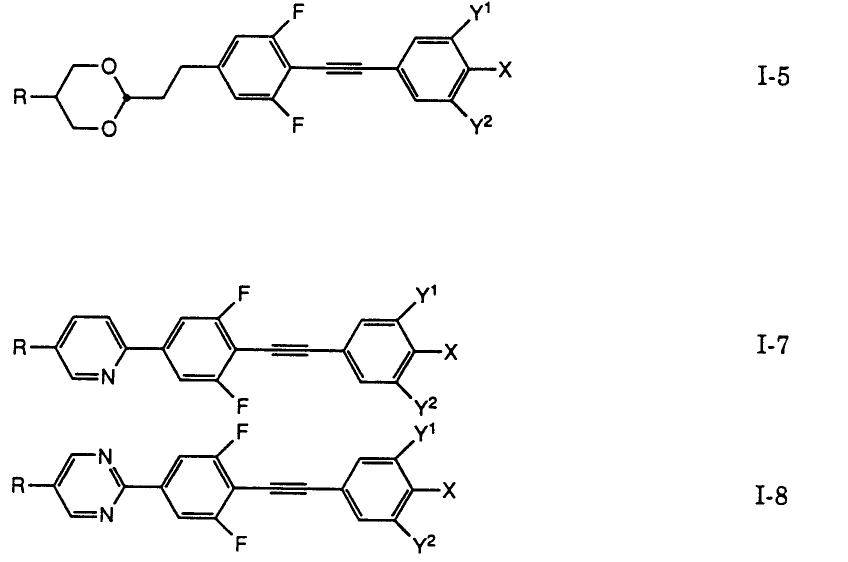 worin
X Fluor, Chlor, Cyano, -CF₃, -OCF₃, -OCHF₂, Alkyl, Alkoxy oder Alkoxyalkyl mit 1 bis 6 Kohlenstoffatomen bedeutet;
Y¹, Y² unabhängig voneinander Fluor oder Wasserstoff bedeuten ; und
R Alkyl, Alkoxy, Alkenyl oder Alkenyloxy mit 1 bzw. 2 bis 12 Kohlenstoffatomen bedeutet, in welchen eine CH₂-Gruppe durch Sauerstoff ersetzt sein kann und/oder eines oder mehrere Wasserstoffatome durch Fluoratome ersetzt sein können.

4. Verbindungen gemäss einem der Ansprüche 1 bis 3, dadurch gekennzeichnet, dass R Alkyl, Alkoxy, Alkenyl oder Alkenyloxy, mit 1 bzw. 2 bis 6 Kohlenstoffatomen, bedeutet, in welchen eine CH₂-Gruppe durch Sauerstoff ersetzt sein kann.

5. Verbindungen gemäss einem der Ansprüche 1 bis 4, dadurch gekennzeichnet, dass einer der Substituenten Y¹ oder Y² Wasserstoff und der andere Wasserstoff oder Fluor bedeutet.

6. Flüssigkristalline Gemische enthaltend mindestens zwei Komponenten, dadurch gekennzeichnet, dass mindestens eine Komponente eine Verbindung der in Anspruch 1 definierten Formel I ist.

7. Flüssigkristalline Gemische gemäss Anspruch 6, dadurch gekennzeichnet, dass der Anteil an Verbindungen der in Anspruch 1 definierten Formel I 3-40 Gew.-% beträgt.

8. Flüssigkristalline Gemische gemäss einem der Ansprüche 6 oder 7, dadurch gekennzeichnet, dass der Anteil an Verbindungen der in Anspruch 1 definierten Formel I 5-30 Gew.-% beträgt.

9. Verwendung der in Anspruch 1 definierten Verbindungen der Formel I für elektro-optische Zwecke.

## Claims (Claims for the following Contracting State(s): CH, FR, GB, IT, LI, NL)

1. Compounds of the general formula 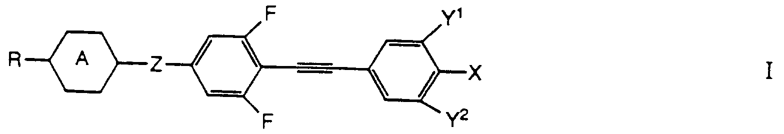 in which
ring A is 1,4-phenylene, pyridine-2,5-diyl, pyrimidine-2,5-diyl, trans-1,4-cyclohexylene- or trans-1,3-dioxane-2,5-diyl;
Z is a single covalent bond, -CH₂CH₂-, -OCH₂-, -CH₂O-, -(CH₂)₄-, -O(CH₂)₃-, -(CH₂)₃O- or, if ring A is a saturated ring, Z is alternatively the transform -CH=CH(CH₂)₂- or -CH=CHCH₂O-;
X is fluorine, chlorine, cyano, -CF₃, -OCF₃, - OCHF₂, alkyl, alkoxy or alkoxyalkyl having 1 to 6 carbon atoms;
Y¹ and Y², independently of one another, are fluorine or hydrogen; and
R is alkyl, alkoxy, alkenyl or alkenyloxy having 1 or 2 to 12 carbon atoms respectively, in which one or two non-adjacent CH₂ groups may be replaced by oxygen and/or one or more hydrogen atoms may be replaced by fluorine atoms.

2. Compounds of the general Formula I according to Claim 1, characterized in that Z is a single covalent bond, -CH₂CH₂- or -CH₂O-.

3. Compounds according to one of Claims 1 and 2 of the general formulae 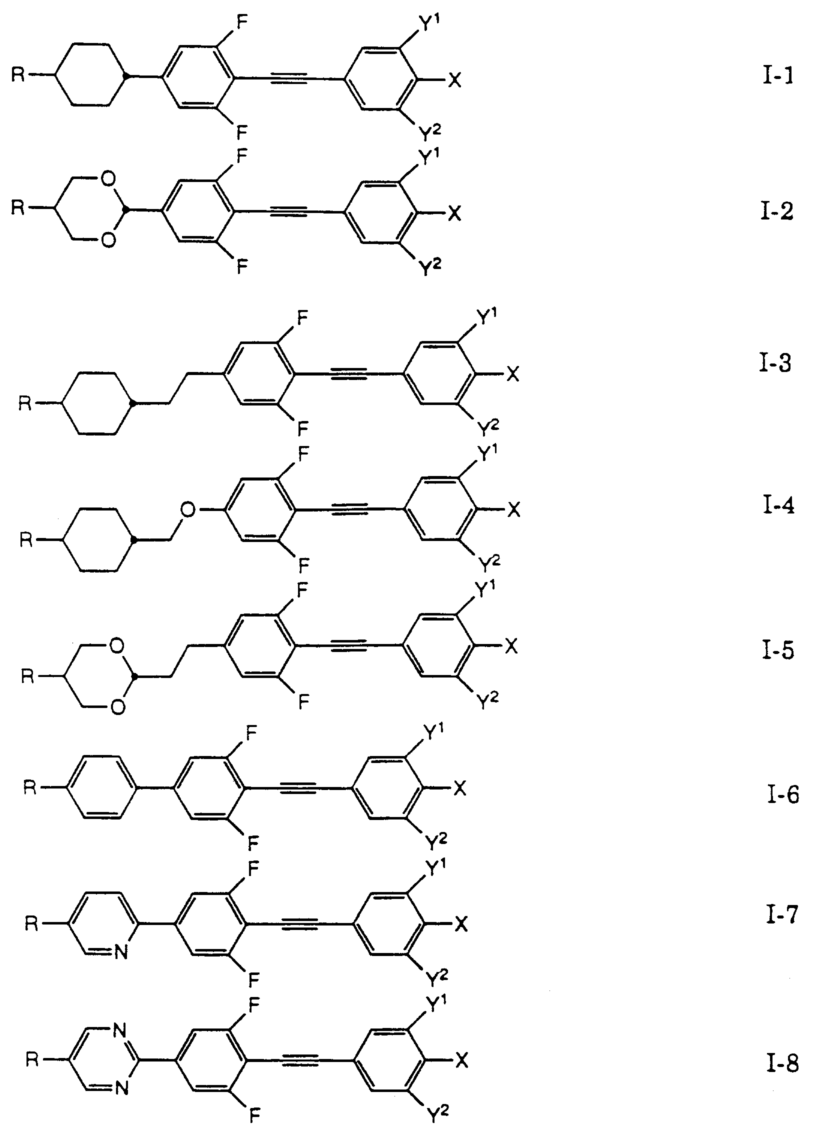 in which
X is fluorine, chlorine, cyano, -CF₃, -OCF₃, - OCHF₂, alkyl, alkoxy or alkoxyalkyl having 1 to 6 carbon atoms;
Y¹ and Y², independently of one another, are fluorine or hydrogen;
R is alkyl, alkoxy, alkenyl or alkenyloxy having 1 or 2 to 12 carbon atoms respectively, in which one CH₂ group may be replaced by oxygen and/or one or more hydrogen atoms may be replaced by fluorine atoms.

4. Compounds according to one of Claims 1 to 3, characterized in that R is alkyl, alkoxy, alkenyl or alkenyloxy having 1 or 2 to 6 carbon atoms respectively, in which one CH₂ group may be replaced by oxygen.

5. Compounds according to one of Claims 1 to 4, characterized in that one of the substituents Y¹ or Y² is hydrogen and the other is hydrogen or fluorine.

6. Liquid-crystalline mixtures comprising at least two components, characterized in that at least one component is a compound of the Formula I defined in Claim 1.

7. Liquid-crystalline mixtures according to Claim 6, characterized in that the proportion of compounds of the Formula I defined in Claim 1 is 3-40% by weight.

8. Liquid-crystalline mixtures according to one of Claims 6 or 7, characterized in that the proportion of compounds of the Formula I defined in Claim 1 is 5-30% by weight.

9. Use of the compounds of the Formula I defined in Claim 1 for electro-optical purposes.

## Claims (Claims for the following Contracting State(s): DE)

1. Compounds of the general formula 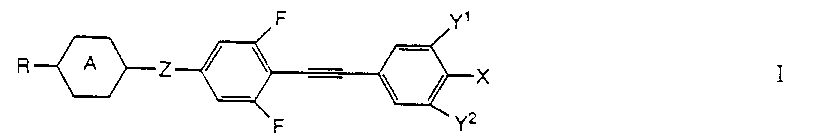 in which
ring A is pyridine-2,5-diyl, pyrimidine-2,5-diyl or trans-1,3-dioxane-2,5-diyl;
Z is a single covalent bond, -CH₂CH₂-, -OCH₂-, -CH₂O-, -(CH₂)₄-, -O(CH₂)₃-, -(CH₂)₃O- or, if ring A is a saturated ring, Z is alternatively the transform -CH=CH(CH₂)₂- or -CH=CHCH₂O-;
X is fluorine, chlorine, cyano, -CF₃, -OCF₃, -OCHF₂, alkyl, alkoxy or alkoxyalkyl having 1 to 6 carbon atoms;
Y¹ and Y² , independently of one another, are fluorine or hydrogen; and
R is alkyl, alkoxy, alkenyl or alkenyloxy having 1 or 2 to 12 carbon atoms respectively, in which one or two non-adjacent CH₂ groups may be replaced by oxygen and/or one or more hydrogen atoms may be replaced by fluorine atoms.

2. Compounds of the general Formula I according to Claim 1, characterized in that Z is a single covalent bond, -CH₂CH₂- or -CH₂O-.

3. Compounds according to one of Claims 1 and 2 of the general formulae 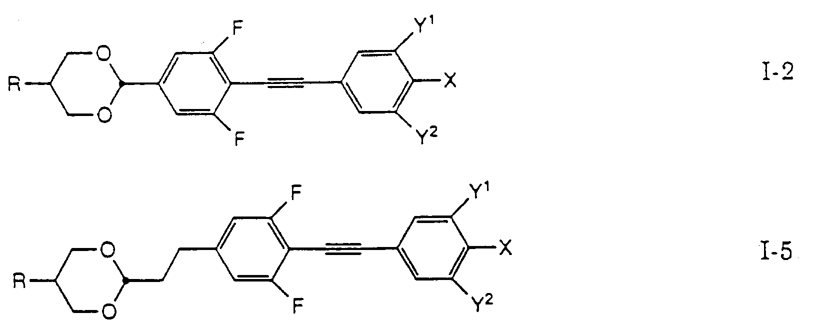 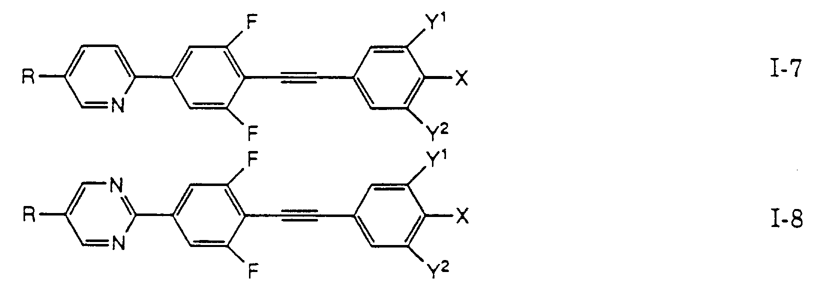 in which
X is fluorine, chlorine, cyano, -CF₃, -OCF₃, - OCHF₂, alkyl, alkoxy or alkoxyalkyl having 1 to 6 carbon atoms;
Y¹ and Y², independently of one another, are fluorine or hydrogen; and
R is alkyl, alkoxy, alkenyl or alkenyloxy having 1 or 2 to 12 carbon atoms respectively, in which one CH₂ group may be replaced by oxygen and/or one or more hydrogen atoms may be replaced by fluorine atoms.

4. Compounds according to one of Claims 1 to 3, characterized in that R is alkyl, alkoxy, alkenyl or alkenyloxy having 1 or 2 to 6 carbon atoms respectively, in which one CH₂ group may be replaced by oxygen.

5. Compounds according to one of Claims 1 to 4, characterized in that one of the substituents Y¹ or Y² is hydrogen and the other is hydrogen or fluorine.

6. Liquid-crystalline mixtures comprising at least two components, characterized in that at least one component is a compound of the Formula 1 in Claim 1.

7. Liquid-crystalline mixtures according to Claim 6, characterized in that the proportion of compounds of the Formula 1 defined in claim 1 is 3-40% by weight.

8. Liquid-crystalline mixtures according to one of Claims 6 or 7, characterized in that the proportion of compounds of the Formula 1 defined in Claim 1 is 5-30% by weight.

9. Use of the compounds of the Formula 1 defined in Claim 1 for electro-optical purposes.

## Revendications (Revendications pour l'(les) Etat(s) contractant(s) suivant(s): CH, FR, GB, IT, LI, NL)

1. Composés de formule générale 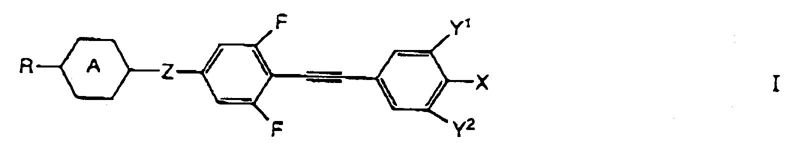 dans laquelle
le cycle A est un cycle 1,4-phénylène, pyridine-2,5-diyle, pyrimidine-2,5-diyle, trans-1,4-cyclohexylène ou trans-1,3-dioxanne-2,5-diyle ;
Z représente une liaison covalente simple, -CH₂CH₂-, -OCH₂-, CH₂O-, (CH₂)₄-, -O(CH₂)₃-, -(CH₂)₃O- ou bien encore, lorsque le cycle A est saturé, la forme trans du groupe -CH=CH(CH₂)₂- ou du -CH=CH CH₂O- ;
X représente le fluor, le chlore, un groupe cyano, -CF₃, -OCF₃, -OCHF₂, alkyle, alcoxy ou alcoxyalkyle contenant un à six atomes de carbone ;
Y¹ et Y² représentent chacun, indépendamment l'un de l'autre, le fluor ou l'hydrogène ; et -
R représenteun groupe alkyle, alcoxy, alcényle ou alcényloxy contenant respectivement un à douze et deux à douze atomes de carbone et dans lesquels un ou deux groupes CH₂ non voisins entre eux peuvent être remplacés par des atomes de fluor.

2. Composés de formule générale I selon la revendication 1, caractérisés en ce que
Z représente une liaison covalente simple, un groupe -CH₂- ou -CH₂O-.

3. Composés selon l'une des revendications 1 et 2, répondant aux formules générales 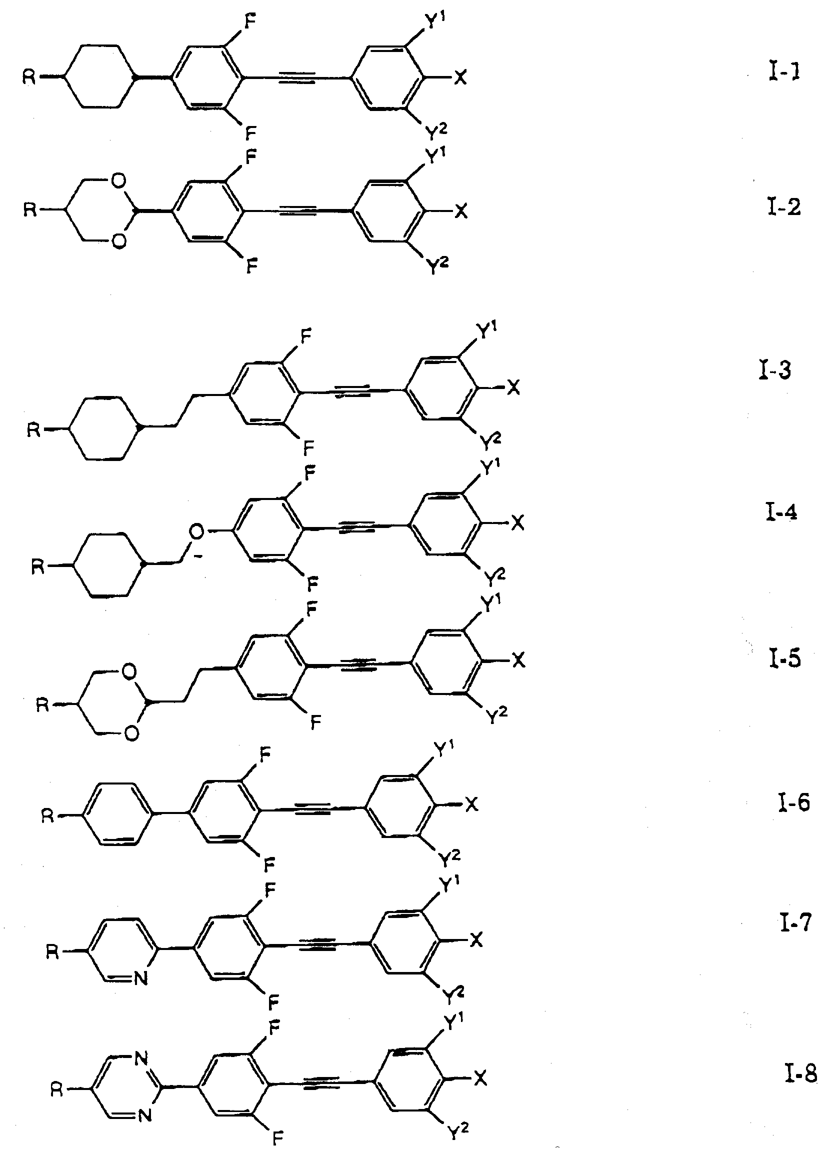 dans lesquelles
X représente le fluor, le chlore, un groupe cyano, -CF₃, -OCF₃, -OCHF₂, alkyle, alcoxy ou alcoxyalkyle en C1-C6 ;
Y¹ et Y² représentent chacun, indépendamment l'un de l'autre, le fluor ou l'hydrogène ;
et
R représente un groupe alkyle, alcoxy, alcényle ou alcényloxy contenant respectivement un à douze et deux à douze atomes de carboneet dans lesquels un groupe CH₂ peut être remplacé par l'oxygène et/ou un ou plusieurs atomes d'hydrogène par des atomes de fluor.

4. Composés selon l'une des revendications 1 à 3, caractérisé en ce que R représente un groupe alkyle, alcoxy, alcényle ou alcynyloxy contenant respectivement un à six et deux à six atomes de carbone et dans lequel un groupe CH₂ peut-être remplacé par l'oxygène.

5. Composés selon une des revendications 1 à 4, caractérisés en ce que l'un des symboles Y¹ et Y² représente l'hydrogène et l'autre l'hydrogène ou le fluor.

6. Mélanges à cristaux liquides contenant au moins deux composants, caractérisés en ce que l'un au moins des composants est un composé de formule I selon la revendication 1.

7. Mélanges à cristaux liquides selon la revendication 6, caractérisés en ce que la proportion des composés de formule I de la revendication est de 3 à 40% en poids.

8. Mélanges à cristaux liquides selon une des revendications 6 ou 7, caractérisés en ce que la proportion des composés de formule I de la revendication 1 est de 5 à 30% en poids.

9. utilisation des composés de formule I selon la revendication 1 dans des apllications électro-optiques.

## Revendications (Revendications pour l'(les) Etat(s) contractant(s) suivant(s): DE)

1. Composés de formule générale 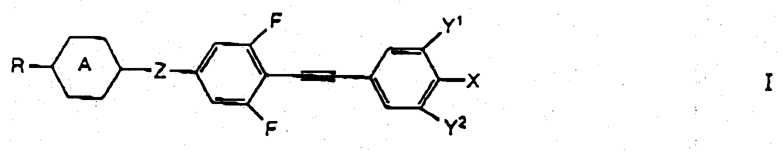 dans laquelle
le cycle A est un cycle pyridine-2,5-diyle, pyrimidine-2,5-diyle ou trans-1,3-dioxanne-2,5-diyle ;
Z représente une liaison covalente simple, -CH₂CH₂-, -OCH₂-, CH₂O-, -(CH₂)₄-, -O(CH₂)₃-, -(CH₂)₃O- ou bien encore, lorsque le cycle A est saturé, la forme trans du groupe -CH=CH(CH₂)₂- ou du -CH=CH CH₂O- ;
X représente le fluor, le chlore, un groupe cyano, -CF₃, -OCF₃, -OCHF₂, alkyle, alcoxy ou alcoxyalkyle contenant un à six atomes de carbone ;
Y¹ et Y² représentent chacun, indépendamment l'un de l'autre, le fluor ou l'hydrogène ; et
R représenteun groupe alkyle, alcoxy, alcényle ou alcényloxy contenant respectivement un à douze et deux à douze atomes de carbone et dans lesquels un ou deux groupes CH₂ non voisins entre eux peuvent être remplacés par des atomes de fluor.

2. Composés de formule générale I selon la revendication 1, caractérisés en ce que
Z représente une liaison covalente simple, un groupe -CH₂- ou -CH₂O-.

3. Composés selon l'une des revendications 1 et 2, répondant aux formules générales 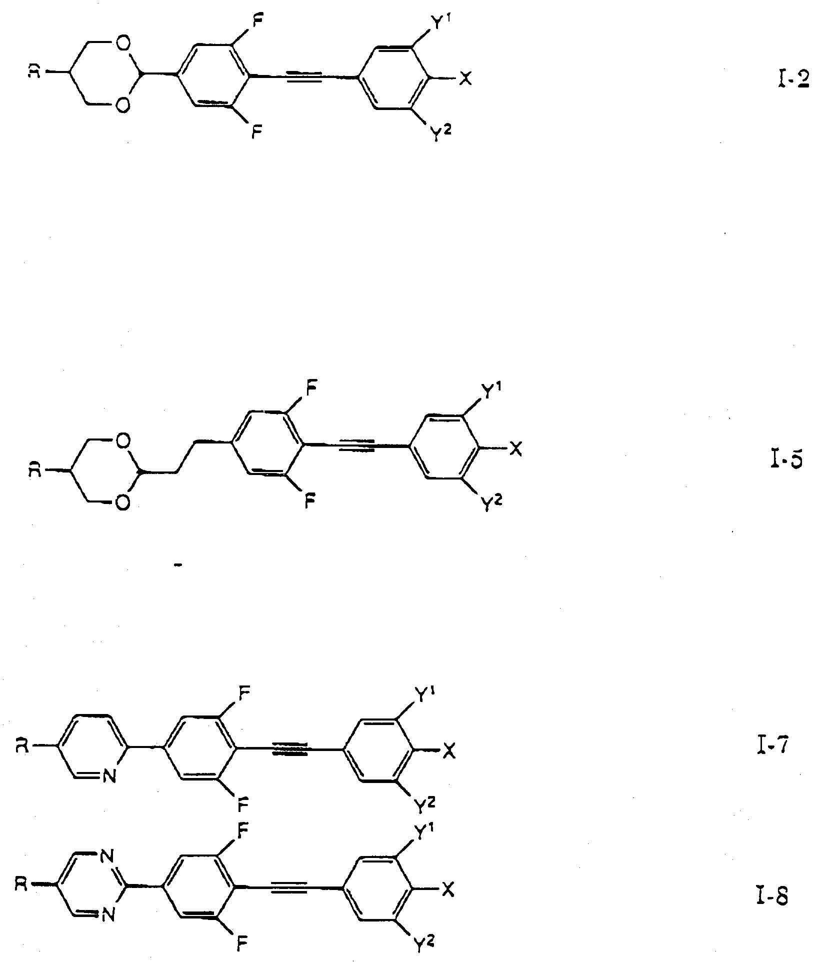 dans lesquelles
X représente le fluor, le chlore, un groupe cyano, -CF₃, -OCF₃, -OCHF₂, alkyle, alcoxy ou alcoxyalkyle en C1-C6 ;
Y¹ et Y² représentent chacun, indépendamment l'un de l'autre, le fluor ou l'hydrogène ;
et
R représente un groupe alkyle, alcoxy, alcényle ou alcényloxy contenant respectivement un à douze et deux à douze atomes de carboneet dans lesquels un groupe CH₂ peut être remplacé par l'oxygène et/ou un ou plusieurs atomes d'hydrogène par des atomes de fluor.

4. Composés selon l'une des revendications 1 à 3, caractérisé en ce que R représente un groupe alkyle, alcoxy, alcényle ou alcynyloxy contenant respectivement un à six et deux à six atomes de carbone et dans lequel un groupe CH₂ peut-être remplacé par l'oxygène.

5. Composés selon une des revendications 1 à 4, caractérisés en ce que l'un des symboles Y¹ et Y² représente l'hydrogène et l'autre l'hydrogène ou le fluor.

6. Mélanges à cristaux liquides contenant au moins deux composants, caractérises en ce que l'un au moins des composants est un composé de formule I selon la revendication 1.

7. Mélanges à cristaux liquides selon la revendication 6, caractérisés en ce que la proportion des composés de formule I de la revendication est de 3 à 40% en poids.

8. Mélanges à cristaux liquides selon une des revendications 6 ou 7, caractérisés en ce que la proportion des composés de formule I de la revendication 1 est de 5 à 30% en poids.

9. utilisation des composés de formule I selon la revendication 1 dans des apllications électro-optiques.
